# EUROPEAN PATENT APPLICATION

(11) **EP 1 510 526 A1**
(43) Date of publication of application: **02.03.2005**
(21) Application number: 03102622.2
(22) Date of filing: 21.08.2003
(51) Int. Cl.: C07K 14/72, A61K 38/00

(54) **Multimeric leptin receptor**

(71) Applicant: VIB vzw, 9052 Zwijnaarde (BE)
(72) Inventor: Tavernier, Jan, 9860 Balegem (BE); Zabeau, Lennart, 9000 Gent (BE)

(57) **Abstract**

The present invention relates to a multimeric leptin receptor, which is at least trimeric, preferably tetrameric, even more preferably homotetrameric. The invention relates further to a domain that is involved in multimerisation of the receptor, and the use of that domain to modulate the signalling of the receptor.

SEQUENCE LISTING

<110>  VIB vzw

<120>  Multimeric leptin receptor

<130>  JTA-LRdom-164

<160>  9

<170>  PatentIn version 3.1

<210>  1
<211>  103
<212>  PRT
<213>  Artificial Sequence

<220>
<223>  Ig-like domain of the leptin receptor

<400>  1

Val Phe Thr Thr Gln Asp Val Val Tyr Phe Pro Pro Lys Ile Leu Thr
1               5                   10                  15

Ser Val Gly Ser Asn Ala Ser Phe His Cys Ile Tyr Lys Asn  Glu Asn
            20                  25                  30

Gln Ile Ile Ser Ser Lys Gln Ile Val Trp Trp Arg Asn Leu Ala Glu
        35                  40                  45

Lys Ile Pro Glu Ile Gln Tyr Ser Ile Val  Ser Asp Arg Val Ser Lys
    50                  55                  60

Val Thr Phe Ser Asn Leu Lys Ala Thr Arg Pro Arg Gly Lys Phe Thr
65                  70                  75                  80

Tyr Asp Ala Val Tyr Cys  Cys Asn Glu Gln Ala Cys His His Arg Tyr
                85                  90                  95

Ala Glu Leu Tyr Val Ile Asp
            100

<210>  2
<211>  51
<212>  DNA
<213>  Artificial Sequence

<220>
<223>  mutagenic oligonucleotide

<400>  2
gttgttttgg gacgatgttt caaactctag aaattgttcc tgggcacaag g                51

<210>  3
<211>  51
<212>  DNA
<213>  Artificial Sequence

<220>
<223>  mutagenic oligonucleotide

<400>  3
ccttgtgccc aggaacaatt tctagagttt gaaacatcgt cccaaaacaa c                51

<210>  4
<211>  44
<212>  DNA
<213>  Artificial Sequence

<220>
<223>  mutagenic oligonucleotide

<400>  4
ggcatatcca atctctcccc ctcgagattt aagttgtttt gtgg                        44

<210>  5
<211>  44
<212>  DNA
<213>  Artificial Sequence

<220>
<223>  mutagenic oligonucleotide

<400>  5
ccacaaaaca acttaaatct cgagggggag agattggata tgcc                        44

<210>  6
<211>  34
<212>  DNA
<213>  Artificial Sequence

<220>
<223>  mutagenic oligonucleotide

<400>  6
gactggagtt cacctcgagt ctttaccaca caag                                   34

<210>  7
<211>  34
<212>  DNA
<213>  Artificial Sequence

<220>
<223>  mutagenic oligonucle otide

<400>  7
cttgtgtggt aaagactcga ggtgaactcc agtc                                    34


<210>  8
<211>  40
<212>  DNA
<213>  Artificial Sequence

<220>
<223>  mutagenic oligonucleotide

<400>  8
gctgaattat acgtgctcga gccgtcaata tcaa tatatc                              40


<210>  9
<211>  40
<212>  DNA
<213>  Artificial Sequence

<220>
<223>  mutagenic oligonucleotide

<400>  9
gatatattga tattgacggc tcgagcacgt ataattcagc                              40

## Description

The present invention relates to a multimeric leptin receptor, which is at least trimeric, preferably tetrameric, even more preferably homotetrameric. The invention relates further to a domain that is involved in the leptin-dependent multimerization of the receptor, and the use of that domain to modulate the signalling of the receptor.

Leptin, the product of the ob gene (1), is a key player in energy homeostasis and body weight control. It is a 16-kDa circulating protein with a structure resembling 4-α-helical bundle cytokines (2). It is mainly secreted by adipose cells, and the circulating level of this hormone strongly correlates with white adipose tissue mass. Leptin regulates energy expenditure and food intake by activating its receptor in certain nuclei of the hypothalamus (3-5). Loss-of-function mutations within the genes for leptin (1, 6), or for its receptor (7-9) cause complex syndromes characterized by morbid obesity, hyperglycemia, hyperinsulinemia, and reduced fertility. Numerous data suggest that leptin also has direct effects on tissues outside the brain, which may help explain its role on basal metabolism, reproduction and hematopoiesis (10-13).

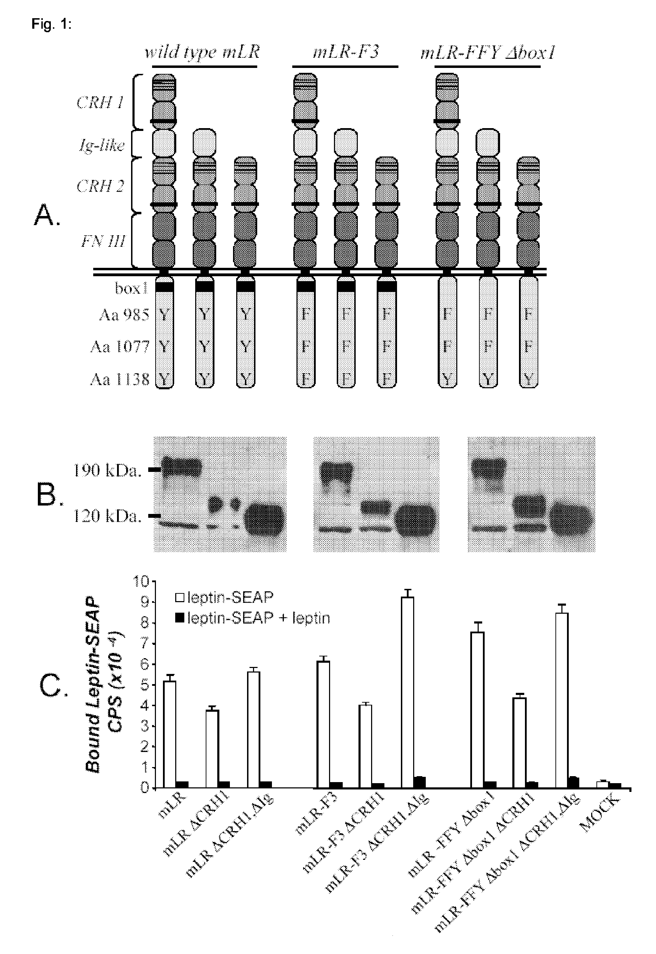

The leptin receptor (LR) is composed of a single subunit, encoded by the db gene (7, 8, 14, 15), and which is a member of the class I cytokine receptor family. It contains two so-called CRH modules, which are formed by two barrel-like domains, each 100 amino acids (aa) in length, and which resemble the fibronectin type III (FN III) and immunoglobulin (Ig) folds. Two conserved disulfide bridges are found in the N-terminal domain, while a WSXWS motif is characteristic for the C-terminal domain. Both LR CRH modules are separated by an Ig-like domain, and are followed by two membrane proximal FN III domains (Fig. 1). Using a panel of deletion and substitution mutants, Fong and co-workers showed that the membrane proximal CRH domain is necessary and sufficient for leptin binding, whereas the two FN III domains are not involved in ligand binding, but are needed for receptor activation (16). Thus far, six isoforms of the LR generated by alternative mRNA splicing have been recognized and termed LRa through LRf. The LR long form (LRIo, or LRb) has an intracellular chain length of 302 aa, and is the only isoform capable of efficient signaling. It is this LRIo isoform that is primarily expressed in specific nuclei of the hypothalamus (17-19), but expression at lower levels in other cell-types has also been observed (20-22). A second isoform, LRa, is a variant lacking most of the cytosolic domain. This LR short form (LRsh) is much more widely expressed, often at higher levels compared to LRIo, e.g. in the choroid plexus, kidney, lung, and liver (23).

Like all members of the class I cytokine receptor family, the LR has no intrinsic kinase activity, and uses cytoplasmic associated Janus kinases (JAKs) for intracellular signaling. Two short conserved sequences in the membrane proximal region (box1 and a putative box2) are thought to mediate binding and activation of the JAK kinases (i.e. JAK2 in the case of the LRIo (24)). In a generally accepted model, leptin binding leads to formation of a receptor complex, allowing activation of JAK2 by cross-phosphorylation. Activated JAK2 then rapidly phophorylates several tyrosine residues in the cytosolic domain of the receptor (i.e. in the case of the mouse LRIo, tyrosines at positions 985, 1077 and 1138). Phosphorylated tyrosine 1138 provides a binding site for STAT3 (Signal Transducers and Activators of Transcription 3) (25). STATs themselves are also substrates for JAKs, and homo- or hetero-dimerize upon phosphorylation, translocate to the nucleus and modulate transcription of target genes. The STAT molecule primarily involved in leptin signaling is STAT3. Leptin-mediated STAT3 activation has been confirmed by Vaisse and co-workers in the hypothalamus of wild type and ob/ob mice after injection of the leptin (26). In cell lines however, leptin mediated STAT1 and STAT5B activation was also shown (27). The other mouse LRIo cytoplasmic tyrosine residues (on position 985 and 1077) are involved in negative feedback of LR signaling, by recruitment of the SH2-containing tyrosine phosphatase-2 (SHP-2) (25, 28), or SOCS-3 (suppressor of cytokine signaling-3) (29-31), or in coupling to other signaling molecules such as mitogen-activated protein kinase (MAPK) (32) and phosphoinositide 3 kinase (PI3K) (33).

It is quite generally accepted that the leptin receptor occurs as a dimer (34, 35). In this model, it has been questioned whether the LR becomes activated upon mere ligand-induced dimerization, i.e. one leptin molecule clusters two receptors chains, or if a pre-existing dimer becomes subject to conformational changes upon ligand binding (34, 35). Although the dimer is the generally accepted model, White and co -workers (34) did not exclude higher order clustering of the leptin receptor. This higher order clustering may help to explain why the LRIo is only to a moderate extent sensitive to the presence of excess LRsh. This short isoform is signaling-deficient, but is often co-expressed with LRIo, accounting for up 95% of LR mRNA in many tissues (7), and may function in leptin clearance or transport through the blood-brain barrier (14). When the LR would be activated upon dimerization and given the inability of LRsh to activate JAKs, only one third of the possible LRIo - LRsh complexes would be able to signal, i.e. LRIo homodimers. In the case of tetramerization, the number of signaling complexes would increase to 60%. Alternatively, differential sorting of the LR short and long isoforms, perhaps due to their different ability to bind JAK2, could also explain this lack of interference (34), and they do not reject the dimeric model. The more recent publication of Couturier and Jockers (35) clearly supports the dimeric model.
Surprisingly, we found that the activated leptin receptor is not dimeric, but multimeric.
Even more surprisingly, we were able to localize a new domain that is essential for the clustering and the activation of the receptor.

It is a first aspect of the invention to provide a multimeric activated leptin receptor complex, comprising at least three receptor subunits. Preferably, said multimeric activated leptin receptor complex is tetrameric, comprising four receptor subunits. Activated as used here, means that the receptor complex is in an active state by ligand binding, and that the signaling pathway is induced. The terms tetrameric and multimeric are only used to indicate the number of receptor molecules in the complex, and do not include the ligand.
Another aspect of the invention is the use of a domain consisting of SEQ ID N°1 to modulate the leptin-dependent multimerization and activation of the leptin receptor. Indeed, according to the invention, this domain plays an essential role in the activation of the receptor, by allowing the higher order oligomerization and consequently the activation of the receptor. Said domain can be used in several ways. It can be used as target for mutagenesis, resulting in an unfunctional domain, whereby the receptor is not longer able to multermerize. Such a receptor functions as a dominant negative mutation. Alternatively, the domain may be presented as soluble peptide, and will disturb the normal ligand receptor interaction. Presenting the soluble domain will therefore inhibit the multimerization and activation of the receptor. Instead of the peptide itself, peptidomimetics can be used. Said domain may also be fused to a leptin antagonist, enhancing the inhibiting action of the antagonist. Leptin-dependent multimerization as used here implies the multimerization step, i.e. the formation of a receptor complex comprising more than 2 receptor subunits, is dependent upon the presence of leptin.
Another aspect of the invention is the use of an antibody directed against a domain consisting of SEQ ID N°1 to modulate the leptin-dependent multimerization and activation of the leptin receptor. It is known to the person skilled in the art how to generate antibodies against a certain domain. Said antibody may be any kind of antibody, including but not limited too polyclonal or a monoclonal antibodies, single chain antibodies, or camelid antibodies. Said antibody will bind to the domain and prevent the multimerization and activation of the receptor. Leptin-dependent multimerization as used here implies the the multimerization step, i.e. the formation of a receptor complex comprising more than 2 receptor subunits, is dependent upon the presence of leptin. Said antibody may be fused to a leptin antagonist, to enhance the inhibiting action of said antagonist.

### BRIEF DESCRIPTION OF THE FIGURES

**Fig. 1.** Expression of wild type LR and LR mutants in HEK293T cells. (A) Schematic representation of the different LR constructs used. Wild type LR, the two signaling-deficient mutants LR-F3 and LR-FFY-Δbox1, and the deletion variants ΔCRH1, and ΔCRH1,Δlg are shown. CRH: Cytokine Receptor Homology module; Ig-like: Immunoglobulin-like domain; FN III: fibronectin type III-like domain. (B) LR Western blot analysis. Lysates of HEK293T cells transfected with 2 µg plasmids encoding (1) LR; (2) LR ΔCRH1; (3) LR ΔCRH1,Δlg; (4) LR-F3; (5) LR-F3 ΔCRH1; (6) LR-F3 ΔCRH1,Δlg; (7) LR-FFY-Δbox1; (8) LR-FFY-Δbox1 ΔCRH1; (9) LR-FFY-Δbox1 ΔCRH 1,Δlg; (10) empty vector, were separated on a 7.5% poly-acrylamide gel, and blotted onto a nitrocellulose membrane. LR and derived mutants were visualized using a polyclonal antibody directed against residues 942 to 953 of the cytoplasmic domain of the receptor, and a horseradish peroxidase labeled secondary antibody. (C) Analysis of LR cell surface expression using a leptin-secreted alkaline phosphatase (SEAP) binding assay. 48 Hours after transfections (same cells as in B), transfected HEK293T cells were incubated with a leptin-SEAP chimeric protein for 90 minutes at room-temperature with NaN₃. As a control for a-specific binding, wild type leptin was added to a final concentration of 2 µg/ml. After inactivation of endogenous phosphatase activity, bound SEAP activity was determined using the CSPD substrate. Bars shown represent mean values, and S.D. values of triplicate measurements. Results are representative for five independent experiments.

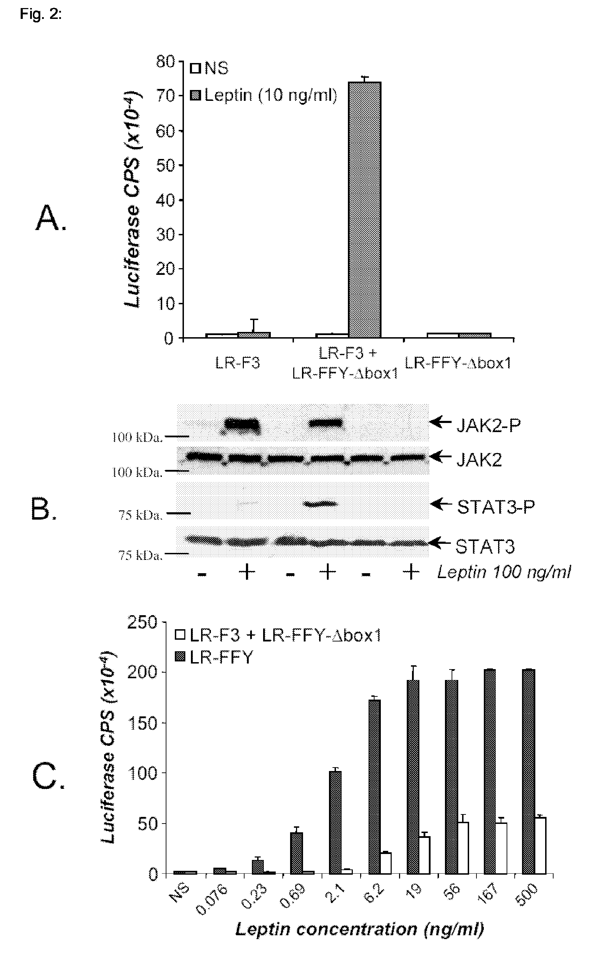

**Fig. 2.** (A) Signaling properties of LR mutants LR-F3 and LR-FFY-Δbox1. Plasmids encoding the LR mutants LR-F3 and LR-FFY-Δbox1 were transiently transfected, individually (2µg) or in combination (1 µg each), in HEK293T cells. The pXP2d2-rPAP-luci reporter construct was co-transfected to measure STAT3 activation. Transfected cells were either stimulated with leptin for 24 hours, or were left un-stimulated (NS). Luciferase measurements were performed in triplicate, and bars and error bars represent mean values and S.D. respectively. Results are representative for at least four independent transfection experiments. (B) JAK2 and STAT3 phosphorylation. Similar transfections were performed to check for phosphorylation of JAK2 and STAT3. Serum-starved cells were stimulated with 100 ng/ml leptin for 10 minutes, or were left unstimulated. Lysates were blotted onto a nitrocellulose membrane and analyzed using phospho-specific antibodies. Total amounts of JAK2 and STAT3 are also shown. (C) Comparison of "wild-type" vs. complementation signaling. HEK293T cells were transfected with either the LR-FFY (2 µg, filled bars), or a combination of the LR-F3 and LR-FFY-Δbox1 mutants (1 µg each, open bars), along with the rPAP-luciferase reporter. Stimulations were with a serial dilution of leptin as indicated. Mean luciferase values and error bars of triplicate measurements are plotted. Results are representative for three independent transfection experiments.

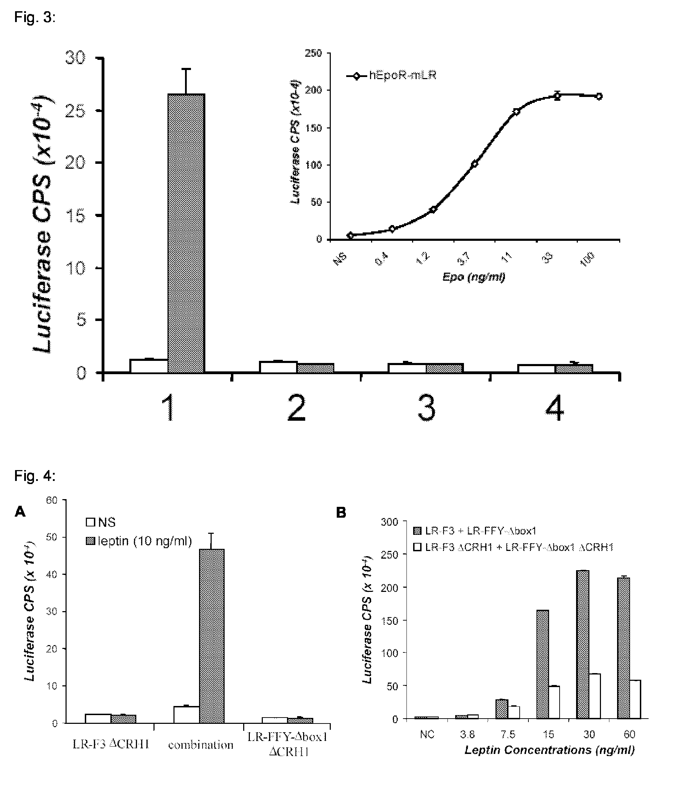

**Fig. 3.** Signaling properties of EpoR-LR chimeric mutants. In order to determine whether complementation occurs when the extracellular part of the LR is replaced by that of the EpoR, plasmids (2 µg) encoding EpoR-LR (1), EpoR-LR-F3 (2), EpoR-LR-FFY-Δbox1 (4), or a combination (1 µg plasmid for each) of the two plasmids encoding the signaling-deficient EpoR chimeric mutants (3), were co-transfected with the rPAP-luciferase reporter in HEK293T cells. Stimulation was with 50 ng/ml Epo (filled bars) or cells were left un-stimulated (open bars). Results presented are the mean values and S.D. for a dataset in triplicate. Inset: In order to measure the biological activity of Epo on cells expressing the EpoR-LR chimera, the receptor was transfected along with the pXP2d2-rPAP-luci reporter construct. Cells were stimulated with a serial dilution of Epo as indicated. Mean luciferase values and error bars of triplicate measurements are plotted.

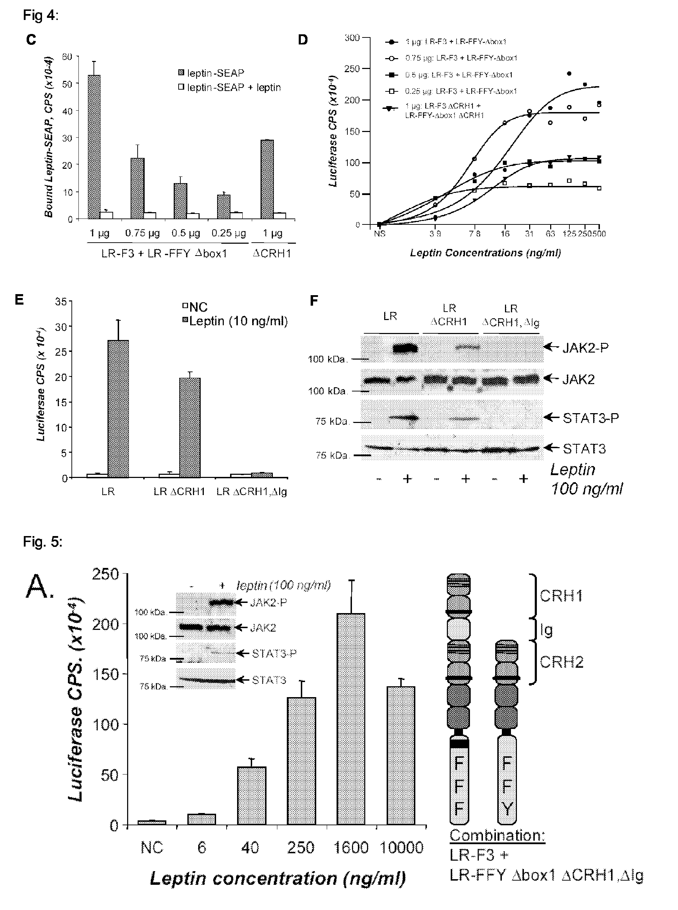

**Fig. 4.** Involvement of CRH1 and Ig-like domains in LR signaling. (A) Signaling via LR-F3 ΔCRH1, and LR-FFY-Δbox1 ΔCRH1. Both mutant receptors were transfected alone (2 µg) or in combination (1 µg each), and transfected HEK293T cells were treated with leptin, or were left un-stimulated (NS). Mean luciferase values and error bars of triplicate measurements are plotted. (B) Deletion of CRH1 leads to a lower leptin signal. Combinations of LR-F3 + LR-FFY-Δbox1 (filled bars) and LR-F3 ΔCRH1 + LR-FFY-Δbox1 ΔCRH1 were transiently transfected in HEK293T cells. Stimulations were overnight with a serial dilution of leptin (as indicated). Mean luciferase values and error bars of triplicate measurements are plotted. (C) Effect of the amount transfected DNA on LR expression. Different quantities of DNA encoding LR-F3 and LR-FFY-Δbox1, or LR-F3 ΔCRH1 and LR-FFY-Δbox1 ΔCRH1 were transfected in HEK293T as indicated. Leptin binding was measured as described in Fig. 1. Bars represent the mean values and S.D. values of triplicate chemiluminescence measurements. (D) Complementation analysis of full-length vs. ΔCRH1 signaling-deficient mutants. HEK293T cells were transfected with 1 µg (filled circles), 0.75 (open circles), 0.5 µg (filled squares), 0.25 µg (open squares) DNA encoding full-length LR-F3 and LR-FFY-Δbox1, or 1 µg (closed triangles) LR-F3 ΔCRH1 and LR-FFY-Δbox1 ΔCRH1. Cells were stimulated overnight with a serial dilution of leptin, as indicated. (E) Role for the LR Ig-like domain in leptin signaling. Signaling via the wild-type LR and two deletion variants thereof, LR ΔCRH1 and LR ΔCRH1,Δlg, were analyzed in HEK293T cells as described. Bars represent mean luciferase counts of triplicate measurements. (F) JAK2 and STAT3 phosphorylation. Phosphorylated and total amounts of JAK2 and STAT3 proteins were determined as described in Fig. 2.

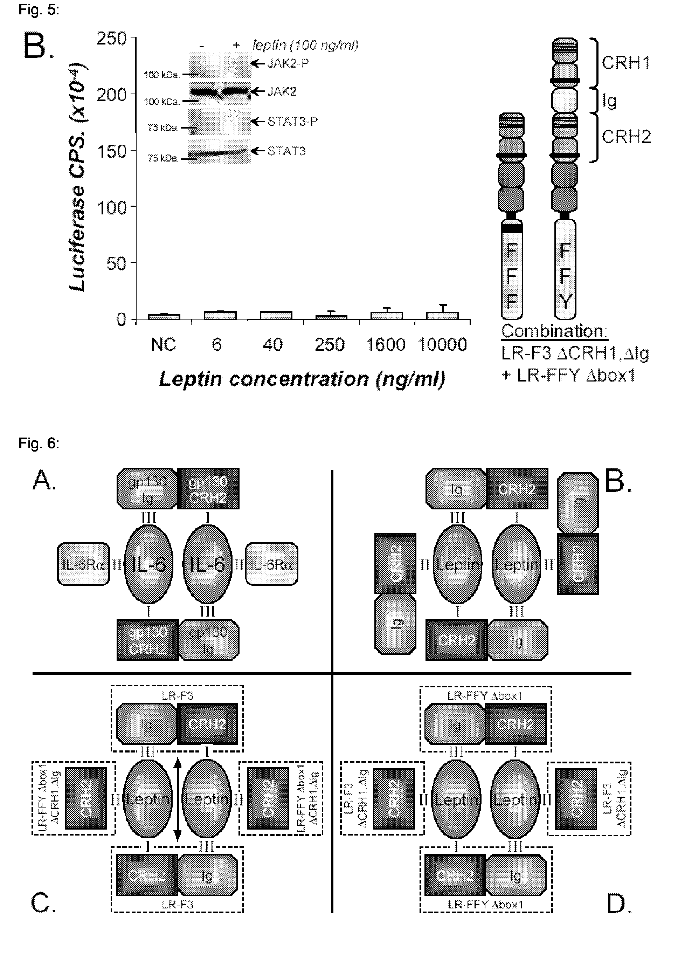

**Fig. 5.** The role of the individual receptor chains in the LR complex is not redundant. HEK293T cells were transfected with the plasmid combinations (each 1 µg transfected) LR-F3 + LR-FFY-Δbox1 ΔCRH1,Δlg (A) and LR-F3 ΔCRH1,Δlg + LR-FFY-Δbox1 (B). The pXP2d2-rPAP-luci reporter construct was co-transfected to measure STAT3 activation. Cells were stimulated overnight with a serial dilution of leptin as indicated. Luciferase measurements were as described earlier, and bars represent mean values. JAK2 and STAT3 phosphorylated and total amounts are shown as insets. Experimental procedures were as described in Fig. 2. A schematic representation of the mutant receptor combinations used is shown on the right. CRH: Cytokine Receptor Homology module; Ig: Immunoglobulin-like domain.
**Fig. 6.** Model for the LR complex. A schematic representation of the IL-6 receptor (A), and of the LR complexes (B) is shown. (C) and (D) accordingly show receptor complexes composed of the LR-F3 + LR-FFY-Δbox1 ΔCRH1,Δlg (C), and LR-F3 ΔCRH1,Δlg + LR-FFY-Δbox1 (D) receptor combination, illustrating the functional asymmetry within the LR complex (see arrow). IL-6Rα: IL-6 receptor α chain; gp130: glycoprotein 130; CRH: Cytokine Receptor Homology module; Ig: Immunoglobulin-like domain; I, an III: putative interaction sites on the ligand.

### EXAMPLES

### Materials and Methods to the examples

### Growth factors and antibodies

Recombinant mouse leptin and human erythropoietin (Epo) were purchased from R&D Systems. Typical stimulations were performed with 100 ng/ml leptin (= 6.25 nM), or 50 ng/ml Epo (= 2.5 nM), unless indicated otherwise.

A peptide was synthesized according to the primary sequence of the mouse LR at position aa. 942 to 953. The KLH-coupled peptides were emulsified in TiterMax Gold Adjuvant (cytRx Corporation) and injected intradermally into New Zealand rabbits. Animals were boosted several times subcutaneously and intramuscularly. The final antisera were passed over Protein-A Sepharose (Amersham-Pharmacia-Biotech) and eluted with 100 mM glycine, 50 mM NaCl, pH 3.0. The IgG fraction was further affinity purified by binding to the corresponding peptide coupled to EAH-Sepharose (Amersham-Pharmacia-Biotech). Final elution was performed with 50 mM diethylamine, 10% glycerol, 50 mM NaCl, pH 11.7.

### Vectors

The pMET7-mouse LR (NCBI Nucleotide accession number U46135) long form (pMET7-mLR) was kindly provided by L. Tartaglia. The mutant leptin receptor LR-F3 was generated as described (31). In brief, the three cytoplasmic tyrosine residues (Y985, Y1077 and Y1138) were mutated to phenylalanines using the Quick Change site-directed mutagenesis procedure (Stratagene). In the second LR variant, LR-FFY-Δbox1, the tyrosines on position 985 and 1077 were mutated to phenylalanines in order to reduce the negative feedback of the signal to a similar level as for the LR-F3. To eliminate JAK activation, two proline residues on position 876 and 878 in the box1 motif, were mutated to serines using the mutagenic oligonucleotides 5'-GTTGTTTTGGGACGATGTTTCAAACTCTAGAAATTGTTCCTGGGCACAAGG-3', and 5'-CCTTGTGCCCAGGAACAATTTCTAGAGTTTGAAACATCGTCCCAAAACA-AC-3'. The LR-FFY variant was constructed as described before (31).

Generation of the EpoR-mLR chimera, containing the extracellular part of EpoR, and the transmembrane and intracellular parts of the mLR, as well as generation of the derived EpoR-LR-F3, has been described previously (61). The EpoR-LR-Δbox1 was constructed in a similar way as the LR-FFY-Δbox1 mentioned above.

LR variants lacking the membrane distal CRH module (Aa 1. to 308) were generated using a site-directed mutagenesis strategy. Using the mutagenic oligonucleotides, 5'-GGCATATCCAATCTCTCCCCCTCGAGATTTAAGTTGTTTTGTGG-3', and 5'-CCACAAAACAACTTAAATCTCGAGGGGGAGAGATTGGATATGCC-3', a Xhol site was introduced at position 90, just after the signal-peptide encoding sequence. A second Xhol site was generated at position 966 (nucleotides 5'-GACTGGAGTTCACCTCGAGTCTTTACCACACAAG-3', and 5'-CTTGTGTGGTA-AAGACTCGAGGTGAACTCCAGTC-3'). The resulting construct was digested with Xhol, and the smallest fragment (228 bp) was ligated back into the opened vector. Deletion of the membrane distal CRH and the neighbouring Ig-like domain (Aa. 309 to 447) was performed along a similar strategy. A third Xhol restriction site was introduced at position 1275 (nucleotides 5'-GCTGAATTATACGTGCTCGAGCCGTCAATATCAAT-ATATC-3' and 5'-GATATATTGATATTGACGGCTCGAGCACGTATAATTCAGC-3'), and the vector was circularised after digestion with Xho I. All constructs were verified by DNA sequence analysis.

Generation of the pXP2d2-rPAP (rat pancreatitis associated protein 1)-luciferase reporter was described before (31). Activation of this reporter is dependent on STAT3, since over-expression of dominant-negative STAT3, but not of dominant-negative STAT1, completely blocks transcriptional activation (62).

### Cell lines and transfection procedures

HEK293T cells were cultured in 10% CO₂ humidified atmosphere at 37°C, and grown using DMEM medium with 4500 mg/l glucose, 10% fetal bovine serum and 50 µg/ml gentamicin (all from Invitrogen).

For transfection experiments, HEK293T cells were freshly seeded and cultured overnight. Cells were transfected overnight with approximately 2 µg (unless stated otherwise) plasmid DNA using a standard calcium phosphate precipitation procedure. One day after transfection, cells were washed with PBS-A (phosphate buffered saline without calcium, magnesium and sodium bicarbonate), and cultured until further use.

### Reporter assay, leptin binding assay, Western blot analysis, and immunoprecipitation

48 Hours after transfection, cells were resuspended with cell dissociation agent (Invitrogen) and seeded in a black 96-well plate (Costar). Cells were stimulated overnight with the appropriate cytokine, and luciferase activity was measured by chemiluminescence. Lysates were prepared (lysis buffer: 25 mM Tris, pH 7.8; 2 mM EDTA; 2 mM DTT; 10% glycerol; 1% Triton X-100), and 35 µl luciferase substrate buffer (20 mM Tricine; 1.07 mM (MgCO₃)₄Mg(OH)₂.5H₂O; 2.67 mM MgSO₄.7H₂O; 0.1 mM EDTA; 33.3 mM DTT; 270 µM Coenzyme A; 470 µM Luciferin; 530 µM ATP; final pH 7.8) was added per 50 µl lysate. Light emission was measured for 5 seconds in a TopCount Chemiluminescence Counter (Packard).

Cell surface expression of *wild type* LR or LR mutants was measured using a binding assay with a mouse leptin-secreted alkaline phosphatase (SEAP) chimeric protein. Generation of this leptin-SEAP has been described before (14). Two days after transfection, cells were washed (wash buffer: DMEM, 0.1% NaN₃, 20 mM Hepes pH 7.0, 0.01% Tween 20) and incubated for 90 minutes at room temperature with a 1/50 dilution of a Cos conditioned medium containing the leptin-SEAP chimera (final concentration: 10 ng/ml). After 3 successive washing steps, cells were lysed (lysis buffer: 1% TritonX-100, 10 mM Tris-HCl pH 7.4). Endogenous phosphatases in the lysates were inactivated (65°C, 30 min), and secreted alkaline phosphatase activity was measured using the CSPD substrate method (PhosphaLight, Tropix) in a TopCount Chemiluminescence Counter (Packard).

Expression of *wild type* LR or LR mutants was also demonstrated using Western blot analysis. Briefly, 4x10⁵ HEK293T cells in a 6-well were transfected with plasmids encoding the LR variants. 60 Hours post transfection, cells were directly lysed in 300 µl loading buffer. After sonication, 40 µl of the lysates were loaded on a 7.5% poly-acrylamide gel. After overnight blotting onto nitrocellulose sheets, LR constructs were revealed using an in-house made polyclonal antibody directed against the intracellular part of the receptor (see above), and a donkey-anti-rabbit horseradish peroxidase coupled antibody.

For STAT3 and JAK2 phosphorylation HEK293T cells were transfected with the appropriate LR variants. 65 Hours later cells were starved in serum-free medium for 5 hours, and were left untreated or stimulated with 100 ng/ml leptin for 15 minutes. Gel electrophoresis and blotting were as described above. STAT3 phosphorylation was checked using the phospho-STAT3-Tyr705 antibody (Cell signaling), according to the manufacturer's guidelines. STAT3 expression levels were verified using an anti-STAT3 antibody (Transduction Laboratories). To detect JAK2 phosphorylation and expression, 0.01 µg pRK5-JAK2 was co-transfected and JAK2 was revealed using an anti-phospho-JAK2 (Y1007, Y1008) antibody (Upstate Biotechnology), or an anti-JAK2 antibody (Upstate Biotechnology).

### Example 1: Construction and expression of LR mutants

To study the requirements for LR activation in more detail, we constructed two types of signaling-deficient LR mutants. In the LR-F3 mutant, all three cytoplasmic tyrosine residues were mutated to phenylalanine, thereby blocking recruitment of STAT3 molecules to the activated receptor. Signaling by the second mutant, LR-FFY-Δbox1, was abolished by two proline to serine mutations at positions 876 and 878 in the box1 motif. These mutations prevent binding and activation of the associated JAK kinases (36). To reduce negative feedback by regulators like SHP-2 or SOCS3, and activation of other signaling pathways such as activation of MAPK and PI3K, we also introduced tyrosine to phenylalanine mutations at positions 985 and 1077. The only intact tyrosine, on position 1138, in this mutant allows activation of and signaling via STAT3. A schematic representation of both mutant receptors is given in Fig. 1 A. Also shown is the expression of both mutants upon transfection in HEK293T cells, using Western blot analysis (Fig. 1 B), and using a binding assay with a leptin-SEAP chimera (Fig. 1 C). Both datasets illustrate that mutations in the cytoplasmic portion of the receptor have no significant effect on the overall expression and cell surface anchoring of the mutant receptors.

To address the role of the membrane distal CRH (also referred to as CRH1) and Ig-like domains in LR signaling, a panel of deletion mutants was constructed. In the first set, we deleted this CRH1 module in the *wild type* LR receptor (LR ΔCRH1) and in both signaling-deficient mutants (LR-F3 ΔCRH1, and LR-FFY-Δbox1 ΔCRH1). Western blot analysis and leptin-SEAP binding data on transfected HEK293T cells illustrate that this deletion results in a minor, but significant decrease in LR cell surface expression (see Fig. 1 B,C). In a similar way, receptors lacking both the CRH1 and Ig-like domains (LR ΔCRH1,Δlg; LR-F3 ΔCRH1,Δlg; and LR-FFY-Δbox1 ΔCRH1,Δlg) were constructed. In contrast, additional deletion of the Ig-like domain may lead to a slightly enhanced LR expression (Fig. 1). Binding of leptin-SEAP was performed at room-temperature, and NaN₃ was added to prevent internalization of the ligand bound receptors. Similar results were obtained when the binding experiment was done on ice (data not shown). It is of note that the expression patterns are in the same range for each set of extracellular deletion mutants, irrespective of the mutations in the cytosolic domains.

### Example 2: Complementation with signaling-deficient LR mutants

We next analyzed leptin-induced signaling via the mutant LR constructs upon (co)-transfection in HEK293T cells. As a read-out, the STAT3-responsive rPAP1 (rat pancreatitis-associated protein 1)-luciferase reporter construct was used as described before (31). Using a substractive cloning strategy, rPAP was identified as late target gene upon leptin stimulation (37). As expected, both LR-F3 and LR-FFY-Δbox1 mutant receptors were inactive, since no STAT3-dependent activation of a rPAP1-luciferase reporter construct, nor STAT3 phosphorylation could be observed (Fig. 2 A). Interestingly, when both receptor mutants were co-expressed, we observed functional complementation leading to a clear increase (up to a 70 fold) of the leptin-induced rPAP-luciferase reporter activation (Fig. 2 A), and a concomitant marked STAT3 phosphorylation (Fig. 2 B). The complementation-dependent signal was less sensitive when compared to the LR-FFY, a control receptor also not subjected to negative feedback (31): approximately 10-fold higher leptin concentrations were required, and a 70% decrease in plateau value was also observed (Fig. 2 C). This is not unexpected since inevitably, co-transfection leads to the formation of several inactive LR complexes (e.g. composed of only one mutant receptor). Importantly, the decrease in sensitivity is limited and all experiments described below were performed using physiologic leptin concentrations (10 ng/ml, or as indicated)

Since two LR-F3 chains are needed for reciprocal activation of JAK kinases, and since at least one LR-FFY-Δbox1 chain with a phosphorylated tyrosine residue is necessary for the recruitment of STATs, our complementation data may be explained by formation of a higher order LR complex.

### Example 3: No complementation observed with EpoR-LR chimeras

It could not *a priori* be excluded that the observed complementation effect with the LR-F3 mutants is due to "mobility" of the JAK kinases, rather thant to a higher order LR complex. This model would imply that upon stimulation of the LR-F3 mutant, the activated JAK kinases might dissociate from the receptor and phosphorylate *in trans* the tyrosine-1138 in the LR-FFY-Δbox1 receptor, leading to subsequent STAT3 activation. In order to exclude this possibility, we analyzed the behavior of EpoR-LR mutants. Several lines of evidence argue for the active state of the EpoR being a dimer: (i) the constitutively active R129C EpoR mutant was found to exist as Epo-independent disulfide-linked dimers when expressed in cells (38). (ii) Biophysical studies identified two interaction sites on Epo for EpoR extracellular domain monomers (39). And (iii) crystal structures of the EpoR extracellular domain in complex with Epo (40), with an Epo-mimetic (41), or with an Epo-antagonistic peptide (42) all showed a 1:2 stoichiometry. We constructed EpoR-LR chimeras containing similar LR-F3 and LR-FFY-Δbox1 mutations, but with the extracellular part of the LR replaced by that of the EpoR. It is generally accepted that the EpoR functions as a homodimer. As observed for the full-length LR mutants, cytoplasmic mutations in the EpoR chimeras did not significantly alter expression, as could be shown by immuno-precipitation with an antibody directed against the extracellular domain of the EpoR, and Western blot analysis with an anti-LR antibody. As shown in Fig. 3, neither EpoR chimera separately, nor in combination, were able to generate a STAT3-dependent signal even at Epo concentration of 50 ng/ml. As a positive control, EpoR-LR, with an intact box1 motif and the critical tyrosine 1138, showed a clear Epo-mediated activation of the rPAP1-luciferase reporter, illustrating that simple dimerization of the LR cytoplasmic domains was sufficient for JAK2 and STAT3 activation. It is of note that stimulation of cells expressing the Epo-LR chimera with 50 ng/ml Epo - the concentration used to test the complementation of signaling - results in an optimal STAT3 dependent signal. Together these data show that receptor dimerization does not allow complementation and lends further support to the LR being a higher order complex.

### Example 4: Role of the CRH1 and Ig-like modules in LR activation and clustering

We next investigated the role of specific sub-domains of the LR extracellular part in LR binding, clustering and activation. Deletion of the CRH1 module in the *wild type* receptor (LR ΔCRH1) results in a limited, but clear decrease in receptor cell surface expression and on leptin binding (both shown in Fig. 1). Like with the full length mutants, the ΔCRH1 deletion in LR-F3 and LR-FFY-Δbox1 mutants did not affect the ability to signal in the complementation assay (Fig. 4 A), excluding a strict requirement for this module in higher order clustering of the LR. However, a lower leptin-induced response was clearly observed when the CRH1 domain was deleted (Fig. 4 B). Similar data were obtained with receptor constructs lacking cytosolic mutations (Fig. 4 E).

We next analyzed whether CRH1 deletion reduced signaling only as a consequence of lowered LR cell-surface expression. In a titration experiment, different amounts of DNA encoding full-length LR-F3 and LR-FFY-Δbox1 (1; 0.75; 0.5 and 0.25 µg each) were co-transfected in HEK293T cells. Leptin-dependent signaling in the transfected cells was measured using the rPAP1-luciferase reporter, and compared to cells co-transfected with 1 µg LR-F3 ΔCRH1 and 1 µg LR-FFY-Δbox1 ΔCRH1. As expected, decreasing the amount of transfected DNA caused reduced leptin binding (Fig. 4 C) and signaling (Fig. 4 D). Clearly, the combination LR-F3 and LR-FFY-Δbox1 (e.g. when 0.75 µg or 0.5 µg DNA were used) showed a significantly higher response when compared to the LR-F3 ΔCRH1 and LR-FFY-Δbox1 ΔCRH1 combination (1 µg transfected), even at lower LR cell surface expression levels. These results indicate that the membrane distal CRH1 also contributes to optimal LR activation.

Interestingly, additional deletion of the Ig-like domain in the *wild type* LR completely abolished STAT3-dependent activation of the rPAP-luciferase reporter (Fig. 4 E). Western blot analysis clearly illustrates that this is due to the inability of the LR ΔCRH1,Δlg variant to activate JAK2, and this in strong contrast to LR and LR ΔCRH1 (Fig. 4 F). Thus far, the function of this Ig-like domain remained unexplored, and was not yet related to LR clustering and/or signaling.

### Example 5: Different roles for receptor chains in an activated LR complex

In order to further define structural requirements for LR activation, different combinations of the two signaling-deficient mutants, and deletion variants thereof, were tested. In Fig. 5, we compared the combined expression of LR-F3 and LR-FFY-Δbox1 ΔCRH1,Δlg with the LR-F3 ΔCRH1,Δlg and LR-FFY-Δbox1 combination. The data showed that leptin-dependent STAT3-activation only occurred when a full-length LR-F3 mutant was expressed (Fig. 5 A). Combined deletion of the CRH1 and Ig-like domains in LR-F3 completely abolished signaling, even at leptin concentrations of 10 µg/ml (Fig. 5 B). As with the *wild type* receptor lacking both CRH1 and Ig-like domain, the lack of rPAP promoter activation can be explained by the total loss of JAK2 phosphorylation and activation. Taken together, these data confirm that the presence of an intact Ig-like module is necessary for signaling, and more specifically for activation of the JAK kinases. Moreover, it appears that Jak2 activation is only possible when the Ig domain and the box1 region are on the same receptor.

### Example 6: The LR complex is a higher order complex

Combined deletion of both the CRH1 and the Ig-like domains in the *wild type* LR led to a complete loss of JAK2 phosphorylation and concomitant STAT3-dependent signaling (Fig. 4 E). Expression and membrane anchoring of the resulting LR mutant is however comparable to that of the full-length receptor (Fig. 1). A role for the Ig-like domain related to ligand binding and receptor activation is well established in the G-CSFR (43, 44), the LIFR (45, 46), and gp130 (47, 48), but was not yet shown for the LR.
Based on (i) the observed complementation data, (ii) the postulated involvement of the Ig-like domain, and (iii) the lack of a strict requirement for CRH1 in signaling, we propose a model wherein leptin functions as a trivalent ligand (Fig. 6 B). Two leptin binding sites (sites I and II) interact with two membrane-proximal CRH2 modules of two distinct LR subunits, and residues belonging to a third site (site III) interact with the Ig-like domain of a third LR chain (Fig. 6 B).
This model strongly resembles that of receptor complexes in the gp130-binding cytokine family. Mutagenesis and binding studies have indicated that these cytokines interact via three distinct binding epitopes with their receptor subunits (49-50). This model was recently supported by data from the crystal structure of Kaposi's sarcoma associated Herpes-virus IL-6 in a complex with the extracellular part of gp130 (47). Two vIL-6 molecules bind to two gp130 CRH2 modules via their site I and to two gp130 Ig-like domains via their site III, leaving the sites II available for interaction with the specific IL-6Rα chains (Fig. 6 A).

This model can help explain the unexpected finding that signaling strongly differs when the combinations LR-F3 + LR-FFY-Δbox1 ΔCRH1,Δlg and LR-F3 ΔCRH1,Δlg + LR-FFY-Δbox1 are compared (Fig. 5). Leptin -induced complex formation in both combinations is most likely identical since the same extracellular receptor parts (full-length + ΔCRH1,Δlg), and similar cytosolic domains are used. According to the model, simultaneous binding of leptin to one LR membrane proximal CRH2 and to the Ig-like domain of a second LR chain leads to clustering of both receptors in such a spatial configuration that the JAKs become properly orientated allowing their cross-phosphorylation and activation (illustrated by the arrow in Fig. 6 C). Much in contrast, recruitment of a JAK-associated receptor (in this case LR-F3 or LR-F3 ΔCRH1,Δlg) by the putative site II, does not lead to JAK activation (Fig. 6 D). This underscores the critical role for the Ig-like domain in JAK activation, and explains why a LR lacking both CRH1 and Ig-like modules is not able to generate a STAT3-dependent signal (Fig. 4 B). This strict requirement for JAK orientation is in marked contrast to STAT activation, since deletion of the CRH1 and Ig-like domains in LR-FFY-Δbox1 has no major effect on signaling. That precise JAK positioning is a prerequisite for efficient signaling is in line with recent reports showing that introduction of 1 to 4 alanine residues in the α-helical transmembrane domain or juxtamembrane intracellular region dramatically affects JAK activation (55, 56). The non-redundant use of individual receptor subunits within a homomeric receptor complex is however a novel and unexpected finding and could so far only be revealed using this complementation strategy. It will be of interest to apply this approach to other receptor systems, including heteromeric cytokine receptors.

### REFERENCES

1. **Zhang Y, Proenca R, Maffei M, Barone M, Leopold L, Friedman JM** 1994 Positional cloning of the mouse obese gene and its human homologue. Nature 372:425-32.
2. **Madej T, Boguski MS, Bryant SH** 1995 Threading analysis suggests that the obese gene product may be a helical cytokine. FEBS Lett 373:13-8.
3. **Halaas JL, Gajiwala KS, Maffei M, Cohen SL, Chait BT, Rabinowitz D, Lallone RL, Burley SK, Friedman JM** 1995 Weight-reducing effects of the plasma protein encoded by the obese gene. Science 269:543-6.
4. **Campfield LA, Smith FJ, Guisez Y, Devos R, Burn P** 1995 Recombinant mouse OB protein: evidence for a peripheral signal linking adiposity and central neural networks. Science 269:546-9.
**5. Pelleymounter MA, Cullen MJ, Baker MB, Hecht R, Winters D, Boone T, Collins F** 1995 Effects of the obese gene product on body weight regulation in ob/ob mice. Science 269:540-3.
6. **Montague CT, Farooqi IS, Whitehead JP, et al.** 1997 Congenital leptin deficiency is associated with severe early-onset obesity in humans. Nature 387:903-8.
**7. Lee GH, Proenca R, Montez JM, Carroll KM, Darvishzadeh JG, Lee JI, Friedman JM** 1996 Abnormal splicing of the leptin receptor in diabetic mice. Nature 379:632-5.
8. **Chen H, Charlat O, Tartaglia LA, et al.** 1996 Evidence that the diabetes gene encodes the leptin receptor: identification of a mutation in the leptin receptor gene in db/db mice. Cell 84:491-5.
9. **Clement K, Vaisse C, Lahlou N, et al.** 1998 A mutation in the human leptin receptor gene causes obesity and pituitary dysfunction. Nature 392:398-401.
10. **Shimabukuro M, Koyama K, Chen G, Wang MY, Trieu F, Lee Y, Newgard CB, Unger RH** 1997 Direct antidiabetic effect of leptin through triglyceride depletion of tissues. Proc Natl Acad Sci U S A 94:4637-41.
11. **Muller G, Ertl J, Gerl M, Preibisch G** 1997 Leptin impairs metabolic actions of insulin in isolated rat adipocytes. J Biol Chem 272:10585-93.
12. **Zachow RJ, Magoffin DA** 1997 Direct intraovarian effects of leptin: impairment of the synergistic action of insulin-like growth factor-I on follicle-stimulating hormone- dependent estradiol-17 beta production by rat ovarian granulosa cells. Endocrinology 138:847-50.
13. **Mikhail AA, Beck EX, Shafer A, et al.** 1997 Leptin stimulates fetal and adult erythroid and myeloid development. Blood 89:1507-12.
14. **Tartaglia LA, Dembski M, Weng X, et al.** 1995 Identification and expression cloning of a leptin receptor, OB-R. Cell 83:1263-71.
15. **Cioffi JA, Shafer AW, Zupancic TJ, Smith-Gbur J, Mikhail A, Platika D, Snodgrass HR** 1996 Novel B219/OB receptor isoforms: possible role of leptin in hematopoiesis and reproduction. Nat Med 2:585-9.
16. **Fong TM, Huang RR, Tota MR, Mao C, Smith T, Varnerin J, Karpitskiy VV, Krause JE, Van der Ploeg LH** 1998 Localization of leptin binding domain in the leptin receptor. Mol Pharmacol 53:234-40.
17. **Mercer JG, Hoggard N, Williams LM, Lawrence CB, Hannah LT, Trayhurn P** 1996 Localization of leptin receptor mRNA and the long form splice variant (Ob-Rb) in mouse hypothalamus and adjacent brain regions by in situ hybridization. FEBS Lett 387:113-6.
18. **Fei H, Okano HJ, Li C, Lee GH, Zhao C, Darnell R, Friedman JM** 1997 Anatomic localization of alternatively spliced leptin receptors (Ob-R) in mouse brain and other tissues. Proc Natl Acad Sci U S A 94:7001-5.
19. **Schwartz MW, Seeley RJ, Campfield LA, Burn P, Baskin DG** 1996 Identification of targets of leptin action in rat hypothalamus. J Clin Invest 98:1101-6.
20. **Hoggard N, Mercer JG, Rayner DV, Moar K, Trayhurn P, Williams LM** 1997 Localization of leptin receptor mRNA splice variants in murine peripheral tissues by RT-PCR and in situ hybridization. Biochem Biophys Res Commun 232:383-7.
21. **Ghilardi N, Ziegler S, Wiestner A, Stoffel R, Heim MH, Skoda RC** 1996 Defective STAT signaling by the leptin receptor in diabetic mice. Proc Natl Acad Sci U S A 93:6231-5.
22. **Dyer CJ, Simmons JM, Matteri RL, Keisler DH** 1997 Leptin receptor mRNA is expressed in ewe anterior pituitary and adipose tissues and is differentially expressed in hypothalamic regions of well- fed and feed-restricted ewes. Domest Anim Endocrinol 14:119-28.
23. **Tartaglia LA** 1997 The leptin receptor. J Biol Chem 272:6093-6
24. **Ghilardi N, Skoda RC** 1997 The leptin receptor activates janus kinase 2 and signals for proliferation in a factor-dependent cell line. Mol Endocrinol 11:393-9.
25. **Carpenter LR, Farruggella TJ, Symes A, Karow ML, Yancopoulos GD, Stahl N** 1998 Enhancing leptin response by preventing SH2-containing phosphatase 2 interaction with Ob receptor. Proc Natl Acad Sci U S A 95:6061-6.
26. **Vaisse C, Halaas JL, Horvath CM, Darnell JE, Jr., Stoffel M, Friedman JM** 1996 Leptin activation of Stat3 in the hypothalamus of wild-type and ob/ob mice but not db/db mice. Nat Genet 14:95-7.
27. **Baumann H, Morella KK, White DW, Dembski M, Bailon PS, Kim H, Lai CF, Tartaglia LA** 1996 The full-length leptin receptor has signaling capabilities of interleukin 6-type cytokine receptors. Proc Natl Acad Sci U S A 93:8374-8.
28. Li C, Friedman JM 1999 Leptin receptor activation of SH2 domain containing protein tyrosine phosphatase 2 modulates Ob receptor signal transduction. Proc Natl Acad Sci U S A 96:9677-82.
29. **Banks AS, Davis SM, Bates SH, Myers MG, Jr.** 2000 Activation of downstream signals by the long form of the leptin receptor. J Biol Chem 275:14563-72.
30. **Bjorbak C, Lavery HJ, Bates SH, Olson RK, Davis SM, Flier JS, Myers MG, Jr.** 2000 SOCS3 mediates feedback inhibition of the leptin receptor via Tyr985. J Biol Chem 275:40649-57.
31. Eyckerman S, Broekaert D, Verhee A, Vandekerckhove J, Tavernier J 2000 Identification of the Y985 and Y1077 motifs as SOCS3 recruitment sites in the murine leptin receptor. FEBS Lett 486:33-7.
32. **Takahashi Y, Okimura Y, Mizuno I, lida K, Takahashi T, Kaji H, Abe H, Chihara K** 1997 Leptin induces mitogen-activated protein kinase-dependent proliferation of C3H10T1/2 cells. J Biol Chem 272:12897-900
33. **Attoub S, Noe V, Pirola L, Bruyneel E, Chastre E, Mareel M, Wymann MP, Gespach C** 2000 Leptin promotes invasiveness of kidney and colonic epithelial cells via phosphoinositide 3-kinase-, rho-, and rac-dependent signaling pathways. Faseb J 14:2329-38
34. **White DW, Kuropatwinski KK, Devos R, Baumann H, Tartaglia LA** 1997 Leptin receptor (OB-R) signaling. Cytoplasmic domain mutational analysis and evidence for receptor homo-oligomerization. J Biol Chem 272:4065-71.
35. **Couturier C, Jockers R** 2003 Activation of the leptin receptor by a ligand-induced conformational change of constitutive receptor dimers. J Biol Chem 278:26604-11.
36. **Bjorbaek C, Uotani S, da Silva B, Flier JS** 1997 Divergent signaling capacities of the long and short isoforms of the leptin receptor. J Biol Chem 272:32686-95
37. **Waelput W, Verhee A, Broekaert D, Eyckerman S, Vandekerckhove J, Beattie JH, Tavernier J** 2000 Identification and expression analysis of leptin-regulated immediate early response and late target genes. Biochem J 348 Pt 1:55-61
38. **Watowich SS, Hilton DJ, Lodish HF** 1994 Activation and inhibition of erythropoietin receptor function: role of receptor dimerization. Mol Cell Biol 14:3535-49
39. **Philo JS, Aoki KH, Arakawa T, Narhi LO, Wen J** 1996 Dimerization of the extracellular domain of the erythropoietin (EPO) receptor by EPO: one high-affinity and one low-affinity interaction. Biochemistry 35:1681-91
40. **Syed RS, Reid SW, Li C, et al.** 1998 Efficiency of signalling through cytokine receptors depends critically on receptor orientation. Nature 395:511-6
41. **Livnah O, Stura EA, Johnson DL, Middleton SA, Mulcahy LS, Wrighton NC, Dower WJ, Jolliffe LK, Wilson IA** 1996 Functional mimicry of a protein hormone by a peptide agonist: the EPO receptor complex at 2.8 A. Science 273:464-71
42. **Livnah O, Johnson DL, Stura EA, et al.** 1998 An antagonist peptide-EPO receptor complex suggests that receptor dimerization is not sufficient for activation. Nat Struct Biol 5:993-1004
43. **Layton JE, Hall NE, Connell F, Venhorst J, Treutlein HR** 2001 Identification of ligand-binding site III on the immunoglobulin-like domain of the granulocyte colony-stimulating factor receptor. J Biol Chem 276:36779-87
44. **Ishibashi M, Tokunaga H, Arakawa T, Tokunaga M** 2001 Expression, purification, and characterization of the active immunoglobulin-like domain of human granulocyte-colony-stimulating factor receptor in Escherichia coli. Protein Expr Purif 21:317-22
45. **Taupin JL, Miossec V, Pitard V, Blanchard F, Daburon S, Raher S, Jacques Y, Godard A, Moreau JF** 1999 Binding of leukemia inhibitory factor (LIF) to mutants of its low affinity receptor, gp190, reveals a LIF binding site outside and interactions between the two cytokine binding domains. J Biol Chem 274:14482-9
**46. Owczarek CM, Zhang Y, Layton MJ, Metcalf D, Roberts B, Nicola NA** 1997 The unusual species cross-reactivity of the leukemia inhibitory factor receptor alpha-chain is determined primarily by the immunoglobulin-like domain. J Biol Chem 272:23976-85
47. **Chow D, He X, Snow AL, Rose-John S, Garcia KC** 2001 Structure of an extracellular gp130 cytokine receptor signaling complex. Science 291:2150-5
48. **Bravo J, Heath JK** 2000 Receptor recognition by gp130 cytokines. Embo J 19:2399-411
49. **Barton VA, Hudson KR, Heath JK** 1999 Identification of three distinct receptor binding sites of murine interleukin-11. J Biol Chem 274:5755-61
50. **Simpson RJ, Hammacher A, Smith DK, Matthews JM, Ward LD 1997** Interleukin-6: structure-function relationships. Protein Sci 6:929-55
51. **Tacken I, Dahmen H, Boisteau O, et al.** 1999 Definition of receptor binding sites on human interleukin-11 by molecular modeling-guided mutagenesis. Eur J Biochem 265:645-55
52. **Inoue M, Nakayama C, Kikuchi K, Kimura T, lshige Y, Ito A, Kanaoka M, Noguchi H** 1995 D1 cap region involved in the receptor recognition and neural cell survival activity of human ciliary neurotrophic factor. Proc Natl Acad Sci U S A 92:8579-83
53. **Di Marco A, Gloaguen I, Graziani R, Paonessa G, Saggio I, Hudson KR, Laufer R** 1996 Identification of ciliary neurotrophic factor (CNTF) residues essential for leukemia inhibitory factor receptor binding and generation of CNTF receptor antagonists. Proc Natl Acad Sci U S A 93:9247-52
54. **Hudson KR, Vernallis AB, Heath JK** 1996 Characterization of the receptor binding sites of human leukemia inhibitory factor and creation of antagonists. J Biol Chem 271:11971-8
55. **Greiser JS, Stross C, Heinrich PC, Behrmann I, Hermanns HM** 2002 Orientational constraints of the gp130 intracellular juxtamembrane domain for signaling. J Biol Chem 277:26959-65
56. **Constantinescu SN, Huang LJ, Nam H, Lodish HF** 2001 The erythropoietin receptor cytosolic juxtamembrane domain contains an essential, precisely oriented, hydrophobic motif. Mol Cell 7:377-85

## Claims

1. Multimeric activated leptin receptor complex, comprising at least three receptor subunits.

2. Multimeric activated leptin receptor according to claim 1, whereby said receptor complex is a tetramer.

3. The use of a domain consisting of SEQ ID N° 1 to modulate leptin-dependent multimerization and activation of the leptin receptor.

4. The use according to claim 3, whereby said domain is fused to a leptin antagonist.

5. The use of an antibody against a domain consisting of SEQ ID N° 1 to inhibit the leptin-dependent multimerization and activation of the leptin receptor.

6. The use according to claim 5, whereby said antibody is fused to a leptin anatgonist.
